(19) Europäisches Patentamt
European Patent Office
Office européen des brevets

(11) Veröffentlichungsnummer: **0 622 365 A1**

(12) # EUROPÄISCHE PATENTANMELDUNG

(21) Anmeldenummer: **94105777.0**

(22) Anmeldetag: **14.04.94**

(51) Int. Cl.$^5$: **C07D 401/04**, C07D 409/04, C07D 413/04, C07D 417/04, C07D 471/04, A61K 31/44, A61K 31/50

(30) Priorität: **27.04.93 DE 4313696**

(43) Veröffentlichungstag der Anmeldung: **02.11.94 Patentblatt 94/44**

(84) Benannte Vertragsstaaten: **AT BE CH DE DK ES FR GB GR IE IT LI LU MC NL PT SE**

(71) Anmelder: **BAYER AG**

**D-51368 Leverkusen (DE)**

(72) Erfinder: **Straub, Alexander, Dr.**
**Moospfad 30**
**D-42113 Wuppertal (DE)**
Erfinder: **Goldmann, Siegfried, Dr.**
**Am Osterholz 91**
**D-423327 Wuppertal (DE)**
Erfinder: **Stoltefuss, Jürgen, Dipl.-Ing.**
**Parkstrasse 20**
**D-42781 Haan (DE)**
Erfinder: **Bechem, Martin, Dr.**
**Hans-Böckler-Strasse 102**
**D-42111 Wuppertal (DE)**
Erfinder: **Gross, Rainer, Prof. Dr.**
**Platzhofstrasse 23**
**D-42115 Wuppertal (DE)**
Erfinder: **Hebisch, Siegbert, Dr.**
**Johann-Breuker-Platz 8**
**D-46244 Bottrop (DE)**
Erfinder: **Hütter, Joachim, Dr.**
**Teschen-Sudberger-Strasse 13**
**D-42349 Wuppertal (DE)**
Erfinder: **Rounding, Howard-Paul, Dr.**
**Pahlkestrasse 15**
**D-42115 Wuppertal (DE)**

(54) **2-Amino-4-heteroaryl-1,4-dihydropyridine, Verfahren zu ihrer Herstellung und ihre Verwendung in Arzneimitteln.**

(57) Die vorliegende Erfindung betrifft neue 2-Amino-4-heteroaryl-1,4-dihydropyridine der allgemeinen Formel (I),

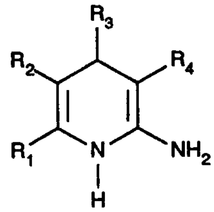

(I)

in welcher $R_1$ bis $R_4$ die in der Beschreibung angegebene Bedeutung haben, Verfahren zu ihrer Herstellung und ihre Verwendung in Arzneimitteln, insbesondere in Mitteln zur Behandlung von Herz-Kreislauferkrankungen.

EP 0 622 365 A1

Rank Xerox (UK) Business Services
(3.10/3.09/3.3.4)

Die vorliegende Erfindung betrifft neue 2-Amino-4-heteroaryl-1,4-dihydropyridine, Verfahren zu ihrer Herstellung und ihre Verwendung in Arzneimitteln, insbesondere in Mitteln zur Behandlung von Herz-Kreislauferkrankungen.

Es ist bereits bekannt, daß einige 2- und 6-Amino-3,4-dihydropyridine neben einer antiarrhythmischen auch eine lipidabsorptionshemmende Wirkung besitzen.

Ferner sind auch 2-Amino-1,4-dihydropyridine mit einer vasodilatorischen und anti-hypertensiven Wirkung beschrieben und andere mit einer positiv inotropen Wirkung und weitgehend neutralem Verhalten gegenüber dem Gefäßsystem bekannt [vgl. EP 515 940]. Aus Publikationen sind ebenfalls 1,4-Dihydropyridine mit positiv inotroper Wirkung bekannt, die in 4-Position durch Heterocyclen substituiert sind [vgl. EP A 450 420].

Bestimmte 4-Thiochromonyl-substituierte 1,4-Dihydropyridine werden von der allgemeinen Beschreibung der EP A 123 095 umfaßt, ohne daß dort konkrete Stoffvertreter genannt werden.

Die vorliegende Erfindung betrifft neue 2-Amino-4-heteroaryl-1,4-dihydropyridine der allgemeinen Formel (I),

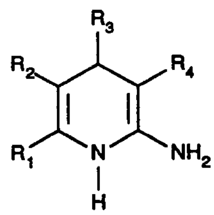

(I)

in welcher

- $R^1$ für geradkettiges oder verzweigtes Alkyl mit bis zu 8 Kohlenstoffatomen steht, das gegebenenfalls durch Hydroxy substituiert ist,
- $R^2$ für geradkettiges oder verzweigtes Alkoxycarbonyl mit bis zu 6 Kohlenstoffatomen steht, das gegebenenfalls durch geradkettiges oder verzweigtes Alkoxy mit bis zu 4 Kohlenstoffatomen substituiert ist,
  für Nitro, Cyano oder Formyl steht,
  oder
- $R^1$ und $R^2$ gemeinsam einen Ring der Formel

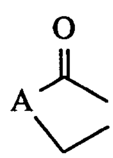

bilden,

worin

- A ein Sauerstoff- oder Schwefelatom, die - $CH_2$- oder -$CH_2CH_2$-Gruppe bedeutet,
- $R^3$ für einen heterocyclischen Arylrest der Formel

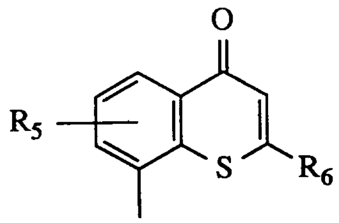 , 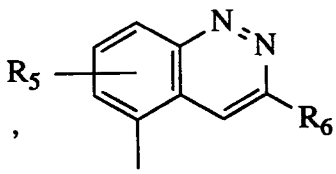 , 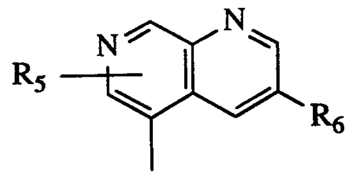

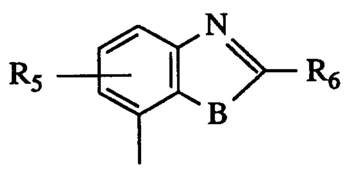 oder 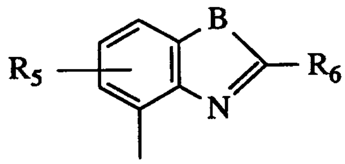

steht,

worin

B ein Sauerstoff- oder Schwefelatom bedeutet,

$R^5$ Wasserstoff, Halogen oder geradkettiges oder verzweigtes Alkyl oder Alkoxy mit jeweils bis zu 8 Kohlenstoffatomen bedeutet,

$R^6$ Aryl mit 6 bis 10 Kohlenstoffatomen bedeutet, das gegebenenfalls bis zu 2-fach gleich oder verschieden durch Halogen, Nitro, Cyano, Trifluormethyl, Trifluormethoxy, Trifluormethylthio, geradkettiges oder verzweigtes Alkyl, Alkoxy oder Alkoxycarbonyl mit jeweils bis zu 8 Kohlenstoffatomen oder Carboxy substituiert ist, oder
geradkettiges oder verzweigtes Alkyl mit bis zu 12 Kohlenstoffatomen bedeutet, oder
Cycloalkyl mit 3 bis 8 Kohlenstoffatomen bedeutet, oder
Pyridyl oder Thienyl bedeutet, das gegebenenfalls durch Halogen substituiert ist,

$R^4$ für eine Gruppe der Formel $-CO-NR^7R^8$, $-CO-D-R^9$ oder $-P(O)(OR^{10})(OR^{11})$ steht, worin

$R^7$ und $R^8$ gleich oder verschieden sind und Wasserstoff oder einen geradkettigen, verzweigten, cyclischen, gesättigten oder ungesättigten Kohlenwasserstoffrest mit bis zu 10 Kohlenstoffatomen bedeuten, der gegebenenfalls durch Cycloalkyl mit 3 bis 8 Kohlenstoffatomen, Halogen, Hydroxy, Cyano oder durch Aryl, Aryloxy, Arylthio mit jeweils 6 bis 10 Kohlenstoffatomen oder durch einen 5-bis 7-gliedrigen, gesättigen oder ungesättigten Heterocyclus mit bis zu 3 Heteroatomen aus der Reihe S, N oder O substituiert ist, wobei die Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder
Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O bedeuten, die gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind, oder

$R^7$ und $R^8$ gemeinsam unter Einbezug des Stickstoffatoms einen 5-bis 8-gliedrigen, gesättigen oder ungesättigten Heterocyclus bilden, der gegebenenfalls durch ein Sauerstoffatom oder durch einen Rest der Formel $S(O)_a$, -CO- oder $-NR^{12}$ unterbrochen ist,
worin

a eine Zahl 0, 1 oder 2 bedeutet,

$R^{12}$ Wasserstoff oder Aryl mit 6 bis 10 Kohlenstoffatomen bedeutet, das gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert ist, oder
einen cyclischen, geradkettigen oder verzweigten, gesättigten oder ungesättigten Kohlenwasserstoffrest mit bis zu 8 Kohlenstoffatomen bedeutet, der gegebenenfalls durch Hydroxy, Halogen oder durch Aryl mit 6 bis 10 Kohlenstoffatomen oder einen 5- bis 7-gliedrigen, gesättigten oder ungesättigten Heterocyclus mit 3 Heteroatomen aus der Reihe S, N oder O substituiert ist, die ihrerseits bis zu 2-fach gleich oder verschieden durch

Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,

und der Heterocyclus gegebenenfalls durch geradkettiges oder verzweigtes Alkoxy oder Alkylthio mit jeweils bis zu 4 Kohlenstoffatomen, Halogen, Aryl mit 6 bis 10 Kohlenstoffatomen, einen 4- bis 7-gliedrigen, gesättigten oder ungesättigten Heterocyclus mit bis zu 3 Heteroatomen aus der Reihe S, N oder O oder durch geradkettiges oder verzweigtes Alkyl mit bis zu 4 Kohlenstoffatomen substituiert ist, das gegebenenfalls seinerseits durch Aryl mit 6 bis 10 Kohlenstoffatomen substituiert sein kann,

D eine direkte Bindung oder ein Sauerstoffatom bedeutet,

$R^9$ Wasserstoff oder Aryl mit 6 bis 10 Kohlenstoffatomen oder einen 4-bis 7-gliedrigen, gesättigten oder ungesättigten Heterocyclus mit bis zu 3 Heteroatomen aus der Reihe S, N oder O bedeutet, die gegebenenfalls bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind, oder einen cyclischen, geradkettigen oder verzweigten, gesättigten oder ungesättigten Kohlenwasserstoffrest mit bis zu 12 Kohlenstoffatomen bedeutet, der gegebenenfalls bis zu 3-fach gleich oder verschieden durch Sauerstoff oder durch -CO-, - CO-NH-, -O-CO-, -CO-O-, -NH-CO-, $-SO_2$-NH-, -NH-$SO_2$-, $-S(O)_b$-oder $-NR^{13}$ unterbrochen ist,

worin

b die oben angegebene Bedeutung von a hat und mit dieser gleich oder verschieden ist,

$R^{13}$ die oben angegebene Bedeutung von $R^{12}$ hat und mit dieser gleich oder verschieden ist,

oder der Kohlenwasserstoffrest gegebenenfalls bis zu 3-fach gleich oder verschieden durch Aryliden mit 6 bis 10 Kohlenstoffatomen oder einen cyclischen Rest der Formel

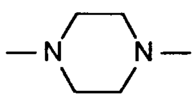 , 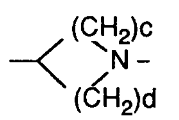 , 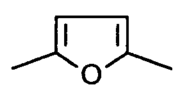 oder 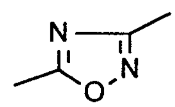

unterbrochen ist,

worin

c und d gleich oder verschieden sind und eine Zahl 1 oder 2 bedeuten,

und worin Aryliden und die Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,

und wobei der Kohlenwasserstoffrest gegebenenfalls bis zu 3-fach gleich oder verschieden durch Cycloalkyl mit 3 bis 8 Kohlenstoffatomen, Halogen, Nitro, Cyano, Hydroxy, $-O-NO_2$, oder durch Aryl, Aryloxy oder Arylthio mit jeweils 6 bis 10 Kohlenstoffatomen oder durch einen 5- bis 7-gliedrigen, gesättigten oder ungesättigen Heterocyclus mit bis zu 3 Heteroatomen aus der Reihe S, N oder O substituiert ist, wobei die Cyclen ihrerseits bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder

der Kohlenwasserstoffrest durch eine Gruppe der Formel $-CO_2-R^{14}$, $-CONR^{15}R^{16}$ oder $-NR^{17}R^{18}$ substituiert ist,

worin

$R^{14}$ die oben angegebene Bedeutung von $R^{12}$ hat und mit dieser gleich oder verschieden ist

und

$R^{15}$, $R^{16}$, $R^{17}$ und $R^{18}$ die oben angegebene Bedeutung von $R^7$ und $R^8$ haben und mit diesen gleich oder verschieden sind,

$R^{10}$ und $R^{11}$ gleich oder verschieden sind und geradkettiges oder verzweigtes Alkyl mit bis zu 8 Kohlenstoffatomen bedeuten,

oder gemeinsam unter Einbezug der Sauerstoffatome einen 5-bis 7-gliedrigen, gesättigen Carbocyclus bilden,

und deren Salze.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Bevorzugt sind Verbindungen der allgemeinen Formel (I),
in welcher

$R^1$ für geradkettiges oder verzweigtes Alkyl mit bis zu 6 Kohlenstoffatomen steht, das gegebenenfalls durch Hydroxy substituiert ist,

$R^2$ für geradkettiges oder verzweigtes Alkoxycarbonyl mit bis zu 4 Kohlenstoffatomen steht, das gegebenenfalls durch geradkettiges oder verzweigtes Alkoxy mit bis zu 3 Kohlenstoffatomen substituiert ist,
für Nitro, Cyano oder Formyl steht,
oder

$R^1$ und $R^2$ gemeinsam einen Ring der Formel

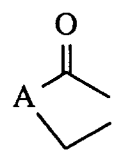

bilden,
worin

A ein Sauerstoff- oder Schwefelatom, die - $CH_2$- oder -$CH_2CH_2$-Gruppe bedeutet,

$R^3$ für einen heterocyclischen Rest der Formel

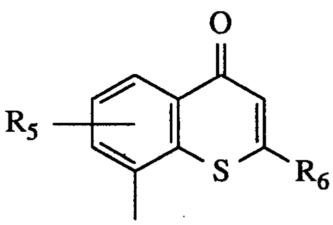 , 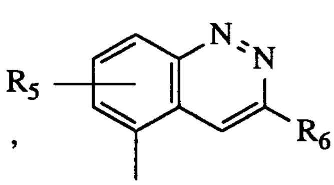 , 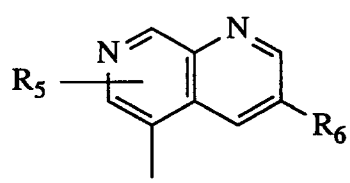

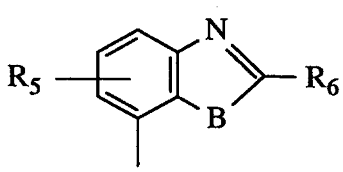 oder 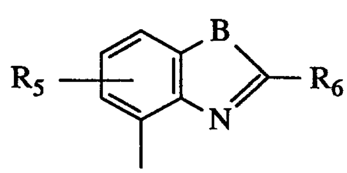

steht,
worin

B ein Sauerstoff- oder Schwefelatom bedeutet,

$R^5$ Wasserstoff, Fluor, Chlor oder geradkettiges oder verzweigtes Alkyl oder Alkoxy mit

jeweils bis zu 2 Kohlenstoffatomen bedeutet,

$R^6$ Phenyl bedeutet, das gegebenenfalls durch Fluor, Chlor, Nitro, Cyano, Trifluormethyl oder durch geradkettiges oder verzweigtes Alkyl oder Alkoxy mit jeweils bis zu 6 Kohlenstoffatomen substituiert ist, oder

geradkettiges oder verzweigtes Alkyl mit bis zu 10 Kohlenstoffatomen bedeutet, oder Cyclopropyl, Cyclohexyl oder Cyclopentyl bedeutet, oder

Pyridyl oder Thienyl bedeutet, das gegebenenfalls durch Fluor, Chlor oder Brom substituiert ist,

$R^4$ für eine Gruppe der Formel $-CO-NR^7R^8$, $-CO-D-R^9$ oder $-P(O)(OR^{10})(OR^{11})$ steht,

worin

$R^7$ und $R^8$ gleich oder verschieden sind und Wasserstoff oder einen geradkettigen, verzweigten, cyclischen, gesättigten oder ungesättigten Kohlenwasserstoffrest mit bis zu 6 Kohlenstoffatomen bedeuten, der gegebenenfalls durch Cyclopropyl, Cyclopentyl, Cyclohexyl, Fluor, Chlor, Brom, Hydroxy oder durch Phenyl oder Pyridyl substituiert ist, wobei die Cyclen ihrerseits durch Fluor, Chlor, Cyano oder durch geradkettiges oder verzweigtes Alkyl, Alkoxy, Alkylthio, Alkoxycarbonyl mit jeweils bis zu 3 Kohlenstoffatomen, Trifluormethyl oder Trifluormethoxy substituiert sein können, oder

Phenyl oder Pyridyl bedeuten, die gegebenenfalls durch Fluor, Chlor oder durch geradkettiges oder verzweigtes Alkyl, Alkoxy, Alkylthio, Alkoxycarbonyl mit jeweils bis zu 4 Kohlenstoffatomen, Trifluormethyl oder Trifluormethoxy substituiert sind,

oder

$R^7$ und $R^8$ gemeinsam unter Einbezug des Stickstoffatoms einen 5-bis 7-gliedrigen, gesättigen oder ungesättigten Heterocyclus bilden, der gegebenenfalls durch ein Sauerstoffatom oder durch einen Rest der Formel $S(O)_a$, - CO- oder $-NR^{12}$ unterbrochen sein kann,

worin

a eine Zahl 0, 1 oder 2 bedeutet,

$R^{12}$ Wasserstoff oder Phenyl bedeutet, oder

einen cyclischen, geradkettigen oder verzweigten, gesättigten oder ungesättigten Kohlenwasserstoffrest mit bis zu 6 Kohlenstoffatomen bedeutet, der gegebenenfalls durch Fluor, Chlor, oder durch Phenyl oder Pyridyl substituiert ist, die ihrerseits durch Fluor, Chlor, Methyl, Ethyl, Methoxy, Ethoxy, Trifluormethyl oder Trifluormethoxy substituiert sein können,

und der Heterocyclus gegebenenfalls durch geradkettiges oder verzweigtes Alkoxy oder Alkylthio mit jeweils bis zu 3 Kohlenstoffatomen, Fluor, Chlor, Phenyl oder Pyridyl oder durch verzweigtes Alkyl mit bis zu 4 Kohlenstoffatomen oder Benzyl substituiert ist,

D eine direkte Bindung oder ein Sauerstoffatom bedeutet,

$R^9$ Wasserstoff, Phenyl oder Pyridyl bedeutet, die gegebenenfalls durch Fluor, Chlor oder durch geradkettiges oder verzweigtes Alkyl, Alkoxy oder Alkylthio mit jeweils bis zu 3 Kohlenstoffatomen, Trifluormethyl oder Trifluormethoxy substituiert sind, oder

einen cyclischen, geradkettigen oder verzweigten, gesättigten oder ungesättigten Kohlenwasserstoffrest mit bis zu 10 Kohlenstoffatomen bedeutet, der gegebenenfalls bis zu 2-fach gleich oder verschieden durch Sauerstoff oder durch - CO-, -O-CO-, -NH-CO-, $-SO_2-NH-$, $-NH-SO_2-$, $-S(O)_b-$ oder $-NR^{13}$ unterbrochen ist,

worin

b die oben angegebene Bedeutung von a hat und mit dieser gleich oder verschieden ist,

$R^{13}$ die oben angegebene Bedeutung von $R^{12}$ hat und mit dieser gleich oder verschieden ist,

oder der Kohlenwasserstoffrest gegebenenfalls bis zu 2-fach gleich oder verschieden durch Phenyliden oder einen cyclischen Rest der Formel

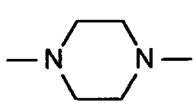 , 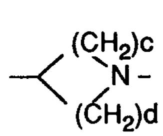 , 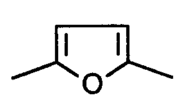 oder 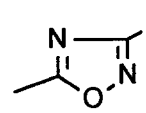

unterbrochen ist,

worin

c und d gleich oder verschieden sind und eine Zahl 1 oder 2 bedeuten,

und wobei der Kohlenwasserstoffrest gegebenenfalls bis zu 2-fach gleich oder verschieden durch Cyclopropyl, Cyclopentyl, Cyclohexyl, Fluor, Chlor, Nitro, Cyano, Hydroxy, - $O\text{-}NO_2$, geradkettiges oder verzweigtes Alkylthio, Alkoxy oder Acyloxy mit jeweils bis zu 6 Kohlenstoffatomen oder durch Phenyl, Phenoxy, Phenylthio oder Pyridyl substituiert ist, wobei die Cyclen ihrerseits bis zu 2-fach gleich oder verschieden durch Fluor, Chlor, Cyano oder durch geradkettiges oder verzweigtes Alkyl, Alkoxy, Alkylthio, Alkoxycarbonyl mit jeweils mit bis zu 4 Kohlenstoffatomen, Trifluormethyl oder Trifluormethoxy substituiert sind, oder

der Kohlenwasserstoffrest durch eine Gruppe der Formel $-CO_2\text{-}R^{14}$, $-CONR^{15}R^{16}$ oder $-NR^{17}R^{18}$ substituiert ist,

worin

$R^{14}$ die oben angegebene Bedeutung von $R^{12}$ hat und mit dieser gleich oder verschieden ist

und

$R^{15}$, $R^{16}$, $R^{17}$ und $R^{18}$ die oben angegebene Bedeutung von $R^7$ und $R^8$ haben und mit diesen gleich oder verschieden sind,

$R^{10}$ und $R^{11}$ gleich oder verschieden sind und geradkettiges oder verzweigtes Alkyl mit bis zu 6 Kohlenstoffatomen bedeuten

oder

$R^{10}$ und $R^{11}$ gemeinsam unter Einbezug des Sauerstoffatoms einen 6-gliedrigen gesättigten Carbocyclus bilden,

und deren Salze,

Besonders bevorzugt sind Verbindungen der allgemeinen Formel (I),

in welcher

$R^1$ für geradkettiges oder verzweigtes Alkyl mit bis zu 4 Kohlenstoffatomen steht, das gegebenenfalls durch Hydroxy substituiert ist,

$R^2$ für geradkettiges oder verzweigtes Alkoxycarbonyl mit bis zu 3 Kohlenstoffatomen oder Methoxyethoxycarbonyl steht, oder

für Nitro, Cyano oder Formyl steht,

oder

$R^1$ und $R^2$ gemeinsam einen Lactonring der Formel

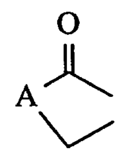

bilden,

worin

A ein Sauerstoff- oder Schwefelatom, die $-CH_2-$ oder $-CH_2CH_2-$Gruppe bedeutet,

$R^3$ für einen heterocyclischen Rest der Formel

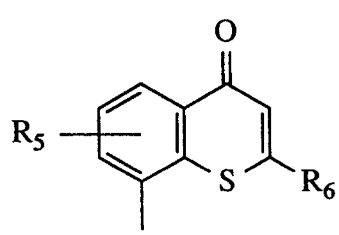
,
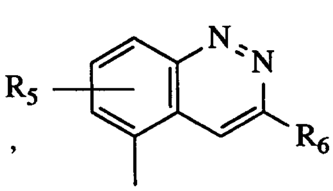
,
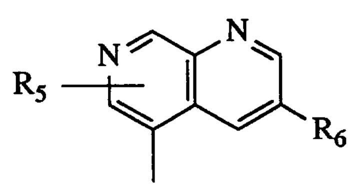

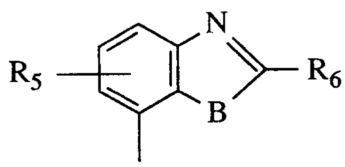 oder 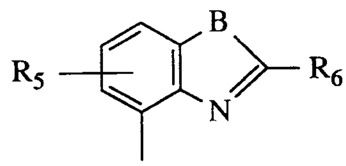

steht,

worin

B — ein Sauerstoff- oder Schwefelatom bedeutet,

$R^5$ — Wasserstoff, Chlor oder Methyl bedeutet,

$R^6$ — Phenyl bedeutet, das gegebenenfalls durch Fluor, Chlor, Nitro, Trifluormethyl oder durch geradkettiges oder verzweigtes Alkyl oder Alkoxy mit jeweils bis zu 4 Kohlenstoffatomen substituiert ist, oder geradkettiges oder verzweigtes Alkyl mit bis zu 8 Kohlenstoffatomen bedeutet, oder
Cyclopropyl, Cyclohexyl oder Cyclopentyl bedeutet, oder Pyridyl oder Thienyl bedeutet, das gegebenenfalls durch Fluor, Chlor oder Brom substituiert ist,

$R^4$ — für eine Gruppe der Formel $-CO-NR^7R^8$ oder $-CO-D-R^9$ steht,
worin

$R^7$ und $R^8$ — gleich oder verschieden sind und Wasserstoff oder Phenyl oder einen geradkettigen, verzweigten, cyclischen, gesättigten oder ungesättigten Kohlenwasserstoffrest mit bis zu 6 Kohlenstoffatomen bedeuten, der gegebenenfalls durch Phenyl substituiert ist, das seinerseits durch Fluor, Chlor, Methyl, Ethyl, Methoxy oder Ethoxy substituiert sein kann,

D — eine direkte Bindung oder ein Sauerstoffatom bedeutet,

$R^9$ — Wasserstoff oder Phenyl bedeutet, oder einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel oder durch einen Rest der Formel -O-CO-, -NH-CO- oder $-NR^{13}$ unterbrochen ist,
worin

$R^{13}$ — Wasserstoff oder geradkettiges oder verzweigtes Alkyl mit bis zu 4 Kohlenstoffatomen bedeutet, das gegebenenfalls durch Phenyl substituiert ist, oder
Phenyl bedeutet,
und wobei der Kohlenwasserstoffrest gegebenenfalls durch Cyclopropyl, Cyclopentyl, Cyclohexyl, Fluor, Chlor, Cyano, Hydroxy, Phenyl, Phenoxy, Phenylthio oder Pyridyl substituiert ist, oder
durch eine Gruppe der Formel $-CO_2-R^{14}$, $-CONR^{15}R^{16}$ oder $-NR^{17}R^{18}$ substituiert ist,
worin

$R^{14}$ — die oben angegebene Bedeutung von $R^{13}$ hat und mit dieser gleich oder verschieden ist
und

$R^{15}$, $R^{16}$, $R^{17}$ und $R^{18}$ — die oben angegebene Bedeutung von $R^7$ und $R^8$ haben und mit diesen gleich oder verschieden sind,

und deren Salze.

Die Herstellung der erfindungsgemäßen Verbindungen der allgemeinen Formel (I) ist dadurch gekennzeichnet, daß man

[A] entweder Aldehyde der allgemeinen Formel (II)

$R^3$-CHO (II)

in welcher

$R^3$ die oben angegebene Bedeutung hat,

direkt mit Verbindungen der allgemeinen Formel (III)

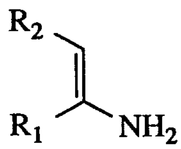

(III)

in welcher

$R^1$ und $R^2$ die oben angegebene Bedeutung haben,

und Verbindungen der tautomeren Formeln (IV) und (IVa)

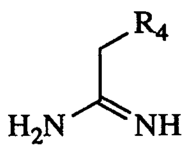

(IV)

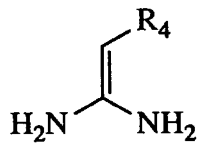

(IVa)

in welcher

$R^4$ die oben angegebene Bedeutung hat,

in inerten Lösemittteln bei Temperaturen zwischen 10 °C und 150 °C umsetzt,

oder

[B] Ylidenverbindungen der allgemeinen Formel (V)

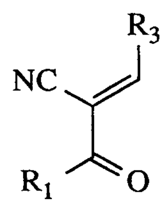

(V)

in welcher

$R^1$ und $R^3$ die oben angegebene Bedeutung haben,

mit Verbindungen der allgemeinen Formel (VI) bzw. (VIa)

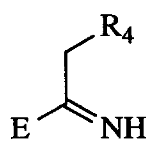

(VI)

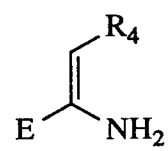

(VIa)

in welcher

$R^4$ die oben angegebene Bedeutung hat, und

E für die Aminogruppe oder für $C_1$-$C_4$-Alkoxy steht,

gegebenenfalls in Anwesenheit von inerten organischen Lösemitteln bei Temperaturen von 10 °C bis 150 °C umsetzt, wobei für den Fall, daß E für $C_1$-$C_4$-Alkoxy steht, Ammoniumsalze, wie Ammoniumacetat zugegeben werden.

Im Fall der reinen Enantiomeren wird entweder das entstehende Diastereomerengemisch der jeweiligen Verbindungen der allgemeinen Formel (I), in welcher $R^2$ für einen definierten chiralen Rest steht, zunächst getrennt, dann in die entsprechenden Carbonsäuren überführt und in einem letzten Schritt verestert oder die jeweiligen Diastereomere werden direkt mit den entsprechenden Alkoholen, insbesondere in Form der Alkoholate umgeestert.

Die erfindungsgemäßen Verfahren können durch folgendes Formelschema beispielhaft erläutert werden:

[A]

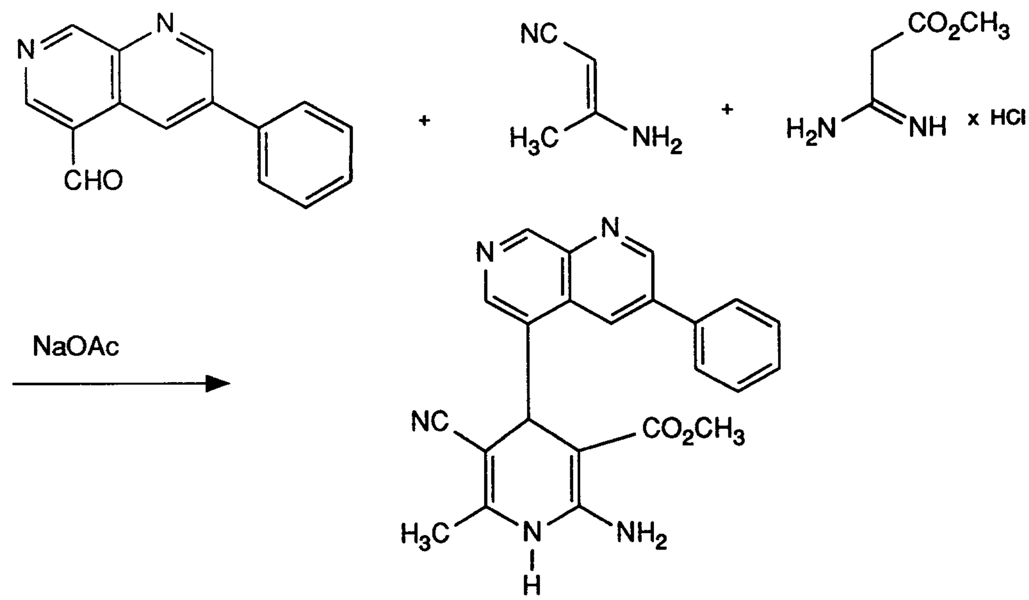


[B]

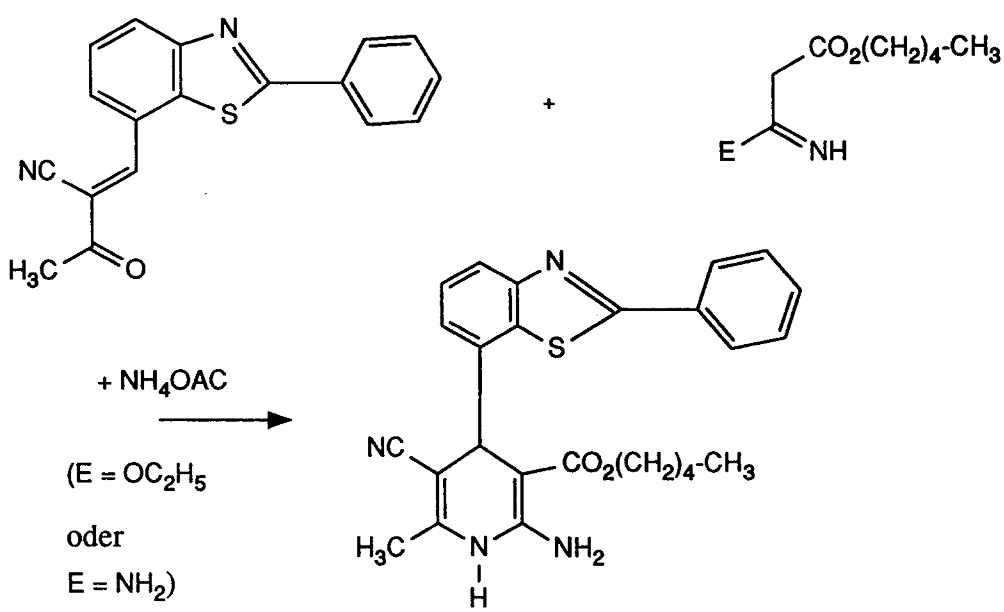


Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure oder halogenierte Kohlenwasserstoffe wie Methylenchlorid, Tetrachlorkohlenstoff oder Kohlenwasserstoffe wie Benzol oder Toluol. Ebenso ist es möglich, Gemische der genannten Lösemittel zu verwenden. Bevorzugt sind in Abhängigkeit von der jeweiligen Verfahrensvariante [A] oder [B] Methanol, Isopropanol, Ethanol und n-Propanol, Acetonitril oder Tetrahydrofuran.

Die Reaktionstemperaturen können in einem größeren Bereich variiert werden. Im allgemeinen arbeitet man zwischen +10 °C und +150 °C, vorzugsweise zwischen +20 °C und +100 °C, insbesondere bei der Siedetemperatur des jeweiligen Lösemittels.

Die Umsetzung kann bei Normaldruck, aber auch bei erhöhtem oder erniedrigtem Druck (z.B. 0,5 bis 3 bar) durchgeführt werden. Im allgemeinen arbeitet man bei Normaldruck.

Geeignet als chirale Esterreste sind alle Ester enantiomerenreiner Alkohole wie beispielsweise 2-Butanol, 1-Phenylethanol, Milchsäure, Milchsäureester, Mandelsäure, Mandelsäureester, 2-Aminoalkohole, Zuckerderivate, Hydroxyaminosäurederivate und viele andere enantiomerenreine Alkohole mehr.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die Aldehyde der allgemeinen Formel (II) sind neu im Fall, daß $R^3$ für die Reste der Formel

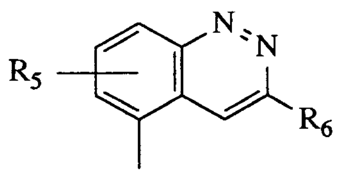

oder

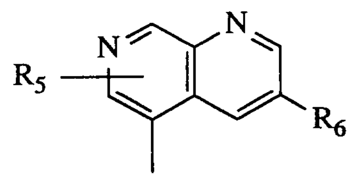

steht,
und können beispielsweise hergestellt werden, indem man

a) substituierte Pyridine der allgemeinen Formel (VII)

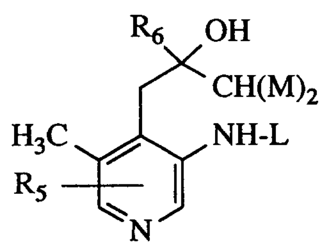

(VII),

in welcher

$R^5$ die oben angegebene Bedeutung hat, vorzugsweise für Chlor steht,

$R^6$ die oben angegebene Bedeutung hat,

L für eine Amino-Schutzgruppe wie beispielsweise tert.Butylcarbonyl steht und

M für geradkettiges oder verzweigtes Alkoxy mit bis zu 4 Kohlenstoffatomen steht,

zunächst mit Protonensäuren, vorzugsweise Salzsäure umsetzt, und mit anschließender Hydrierung zu den Verbindungen der allgemeinen Formel (VIII)

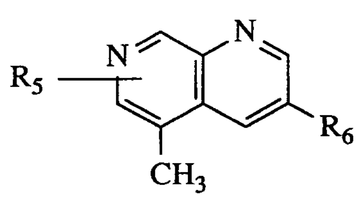

(VIII),

in welcher

$R^5$ und $R^6$ die oben angegebene Bedeutung haben,

in inerten Lösemitteln cyclisiert und in einem letzten Schritt in einem organischen Lösemittel oder Naphthalin, vorzugsweise Naphthalin, die Methylgruppe bevorzugt mit Selendioxid oxidiert

und im Fall, daß $R^3$ für den Rest der Formel

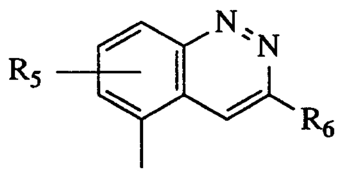

steht,

b) Verbindungen der allgemeinen Formel (IX)

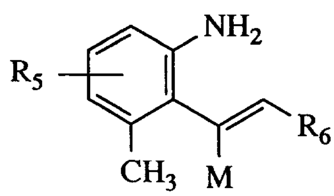

(IX),

in welcher

$R^5$, $R^6$ und M die oben angegebene Bedeutung haben,

über die diazotierte Stufe ($NH_2$ -> $N_2^+$) zu Verbindungen der allgemeinen Formel (X)

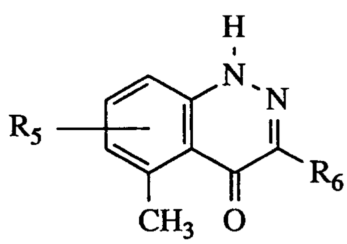

(X),

in welcher

$R^5$ und $R^6$ die oben angegebene Bedeutung haben,

cyclisiert, in einem zweiten Schritt mit $PCl_5/POCl_3$ in Verbindungen der allgemeinen Formel (XI)

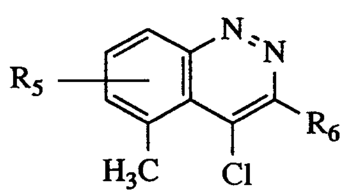

(XI),

in welcher

$R^5$ und $R^6$ die oben angegebene Bedeutung haben,

überführt, hydriert und abschließend die Methyl-Gruppe in inerten Lösemitteln oxidiert.

Die Aldehyde der allgemeinen Formel (II), in welcher

$R^3$ für die Reste der Formel

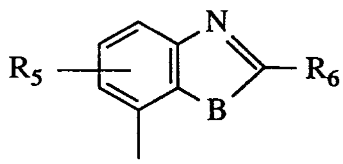 , 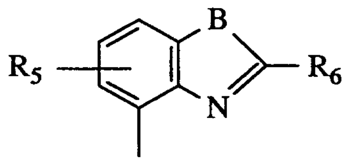 oder 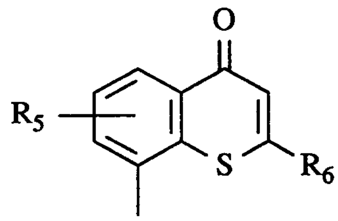

steht,
sind bekannt oder können durch übliche Methoden hergestellt werden.

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether oder Amide wie Hexamethylphosphorsäuretriamid oder Dimethylformamid, oder Essigsäure sowie Methylenchlorid, Tetrachlorkohlenstoff oder Toluol. Ebenso ist es möglich, Gemische der genannten Lösemittel zu verwenden.

Die Oxidation der Verbindungen der allgemeinen Formeln (VIII) und (X) erfolgt im allgemeinen mit Oxidationsmitteln wie beispielsweise Chromylchlorid, Cerammoniumnitrat, Silber(II)oxid, Selendioxid oder ein Chrom(VI)oxid in Verbindung mit Essigsäureanhydrid. Bevorzugt ist Selendioxid.

Die Oxidationen kann bei Normaldruck oder erhöhtem oder erniedrigtem Druck (beispielsweise von 0,5 bis 5 bar),
vorzugsweise bei Normaldruck, durchgeführt werden.

Als Basen eignen sich für die einzelnen Verfahrensstufen die üblichen Alkali-und Erdalkalihydroxide und -carbonate, vorzugsweise Natriumhydroxid und Natriumhydrogencarbonat.

Die Verbindungen der allgemeinen Formeln (VIII), (X) und (XI) sind neu und können nach dem oben aufgeführten Verfahren hergestellt werden.

Die Verbindungen der allgemeinen Formel (VII) sind neu und können hergestellt werden, indem man die S-Nitrogruppe in der bekannten Verbindung 2-Chlor-3,4-dimethyl-5-nitropyridin, zunächst zu der entsprechenden 5-Aminogruppe nach üblichen Methoden, beispielsweise durch Hydrierung mit $H_2$/Pd/C in Dioxan, reduziert, anschließend durch Umsetzung mit Pivaloylchlorid die Aminogruppe blockiert, intermediär mit n-Butyllithium in Tetrahydrofuran deprotoniert und im einem letzten Schritt mit 2,2-Dialkoxyacetophenonen umsetzt.

Die Verbindungen der allgemeinen Formeln (III), (IV), (IVa), (V), (VI), (VIa) und (IX) sind an sich bekannt oder können nach üblichen Methoden hergestellt werden.

Die vorstehenden Herstellungsverfahren sind lediglich zur Verdeutlichung angegeben. Die Herstellung der Verbindungen der Formel (I) ist nicht auf diese Verfahren beschränkt, sondern jede Modifikation dieser Verfahren ist in gleicher Weise für die Herstellung der erfindungsgemäßen Verbindungen anwendbar.

Die erfindungsgemäßen Verbindungen zeigen ein nicht vorhersehbares, wertvolles pharmakologisches Wirkspektrum. Sie beeinflussen die Kontraktionskraft des Herzens und den Tonus der glatten Muskulatur.
Sie können deshalb in Arzneimitteln zur Beeinflussung des pathologisch veränderten Blutdrucks, als Koronartherapeutika und zur Behandlung der Herzinsuffizienz eingesetzt werden. Darüber hinaus können sie zur Behandlung von Herzrhythmusstörungen, zur Senkung des Blutzuckers, zur Abschwellung von Schleimhäuten und zur Beeinflussung des Salz- und Flüssigkeitshaushaltes verwendet werden.

Die Herz- und Gefäßwirkungen wurden am isoliert perfundierten Herzen des Meerschweinchens gefunden. Dazu werden die Herzen von 250 bis 350 g schweren Meerschweinchen verwendet. Die Tiere werden mit einem Schlag auf den Kopf getötet, der Thorax geöffnet, und in die freipräparierte Aorta eine Metallkanüle eingebunden. Das Herz wird mit den Lungen aus dem Thorax herausgetrennt und über eine Aortenkanüle an die Perfusionsapparatur bei laufender Perfusion angeschlossen. Die Lungen werden an den Lungenwurzeln abgetrennt. Als Perfusionsmedium dient eine Krebs-Henseleit-Lösung (118,5 mmol/l NaCl, 4,75 mmol/l KCl, 1,19 mmol/l $KH_2PO_4$, 1,19 mmol/l $MgSO_4$, 25 mmol/l $NaHCO_3$, 0,013 mmol/l $Na_2EDTA$), deren $CaCl_2$-Gehalt 1,2 mmol/l beträgt. Als energielieferndes Substrat werden 10 mmol/l Glucose zugesetzt. Vor der Perfusion wird die Lösung partikelfrei filtriert. Die Lösung wird mit Carbogen (95% $O_2$, 5% $CO_2$) zur Aufrechterhaltung des pH-Wertes 7,4 begast. Die Herzen werden mit konstantem Fluß (10 ml/min) bei 32 °C mittels einer Rollenquetschpumpe perfundiert.

Zur Messung der Herzfunktion wird ein flüssigkeitsgefüllter Latexballon, der über eine Flüssigkeitssäule mit einem Druckaufnehmer verbunden ist, durch den linken Vorhof in den linken Ventrikel eingeführt, und die isovolumetrischen Kontraktionen auf einem Schnellschreiber registriert. Der Perfusionsdruck wird mittels

eines Druckaufnehmers, der vor dem Herzen mit dem Perfusionssystem in Verbindung steht, registiert. Unter diesen Bedingungen zeigt eine Senkung des Perfusionsdrucks eine Koronardilatation, eine Zu- bzw. Abnahme der linksventrikulären Kontraktionsamplitude eine Senkung bzw. einen Anstieg der Herzkontraktilität an. Die erfindungsgemäßen Verbindungen werden in geeigneten Verdünnungen in das Perfusionssystem kurz vor dem isolierten Herzen perfundiert.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Beispiel 1

2-Amino-5-cyano-6-methyl-4-(3-phenyl-1,7-naphthyridin-5-yl)-1,4-dihydropyridin-3-carbonsäureethylester

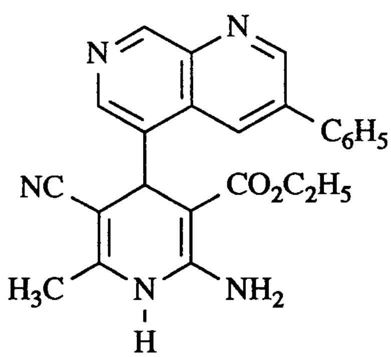

2 g (8,5 mmol) 3-Phenyl-1,7-naphthyridin-5-carboxaldehyd werden in 20 ml Ethanol suspendiert und mit 0,7 ml (8,5 mmol) 5-Methylisoxazol verrührt. Es wird eine Lösung von 196 mg Natrium in 14 ml Ethanol zugegeben und 2 Stunden bei 50 °C gerührt. Es werden 1,42 g Amidinoessigsäureethylester-hydrochlorid und 0,51 ml (8,5 mmol) Essigsäure zugegeben und 16 Stunden gekocht. Nach Erkalten werden 10 g Kieselgel zugegeben und im Vakuum eingeengt. Der Rückstand wird auf einer Kieselgelsäule mit Toluol/Essigester-Gemischen chromatographiert. Nach Einengen der reinen Fraktionen wird durch Verreiben mit Ether kristallisiert. Man erhält 2 g Kristalle.

Beispiel 2

2-Amino-5-cyano-6-methyl-4-(3-(4-fluorphenyl)-1,7-naphthyridin-5-yl)-1,4-dihydropyridin-3-carbonsäureisopropylester

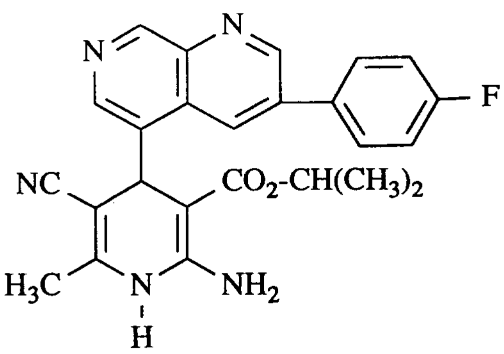

0,5 g (1,98 mmol) 3-(4-Fluorphenyl)-1,7-naphthyridin-5-carboxaldehyd werden in 20 ml Isopropanol gelöst und mit 0,161 ml 5-Methylisoxazol verrührt. Es wird eine Lösung von 46 mg Natrium in 5 ml Isopropanol zugegeben und 2 Stunden bei 50 °C gerührt. Es werden 358 mg (1,98 mmol) Amidinoessigsäureisopropylesterhydrochlorid und 0,113 ml Eisessig zugegeben und 15 h gekocht. Nach Erkalten gibt man 3 g Kieselgel hinzu und engt im Vakuum ein. Der Rückstand wird auf einer Kieselgelsäule mit Toluol/Essigester-Gemischen chromatographiert. Nach Einengen der reinen Fraktionen wird durch Verreiben mit Ether kristallisiert. Man erhält 104 mg Kristalle.

In Analogie zu den Vorschriften der Beispiele 1 und 2 werden die in Tabelle 1 und 2 aufgeführten Verbindungen hergestellt:

Tabelle 1:

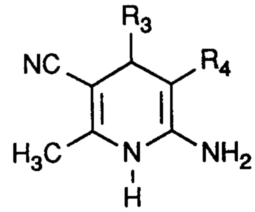

| Bsp.-Nr. | $R^3$ | $R^4$ | F°C |
|---|---|---|---|
| 3 | 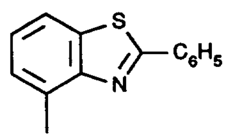 | $-CO_2-C_2H_5$ | 232 |
| 4 | 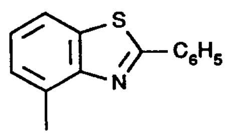 | $-CO_2-CH(CH_3)_2$ | 233 |
| 5 | 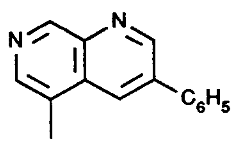 | $-CO_2(CH_2)_2CH_3$ | 239 |
| 6 | 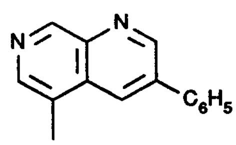 | $-CO_2CH(CH_3)_2$ | 250 |
| 7 | 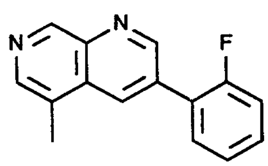 | $-CO_2CH(CH_3)_2$ | 226 |
| 8 | 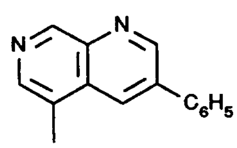 | $-CO_2C_2H_5$ | 265 |

Tabelle 2:

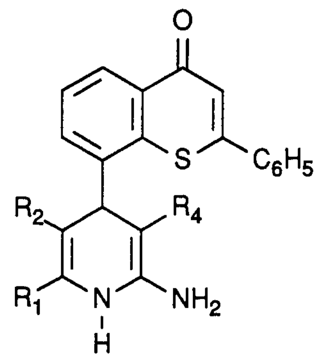

| Bsp.-Nr. | $R^1$ | $R^2$ | $R^4$ | F°C | Enantiomer/Diastereomer/Drehwert |
|---|---|---|---|---|---|
| 9 | 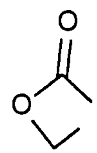 | | $-CO_2-C_2H_5$ | | |
| 10 | $-CH_2-OH$ | $-CO_2-C_2H_5$ | $-CO_2-C_2H_5$ | | |
| 11 | $-CH_3$ | -CN | $-CO_2-C_2H_5$ | | |
| 12 | $-CH_3$ | -CN | $-CO_2-CH(CH_3)_2$ | | |
| 13 | $-CH_3$ | -CN | $-CO_2-C_2H_5$ | 243 (Zers.) | (+) $[\alpha]_D$ = +224,44 (c=0,59/$CHCl_3$) |
| 14 | $-CH_3$ | -CN | $-CO_2-CH(CH_3)_2$ | 243 (Zers.) | (+) $[\alpha]_D$ = + 225,52 (c=0,535/$CHCl_3$) |
| 15 | $-CH_3$ | -CN | $-CO_2CH(CH_3)_2$ | 256 (Zers.) | $[\alpha]_D$ = -219,45 (c=0,851/$CHCl_3$) |

Fortsetzung Tabelle 2:

| Bsp.-Nr. | $R^1$ | $R^2$ | $R^4$ | F°C | Enantiomer/Diastereomer/Drehwert |
|---|---|---|---|---|---|
| 16 | $-CH_3$ | -CN | 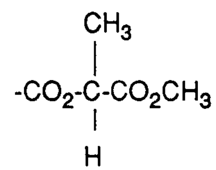 | | (+) $[\alpha]_D$ = 229,75 ($c$=0,7735/$CHCl_3$) — Diasteromere |
| 17 | $-CH_3$ | -CN | 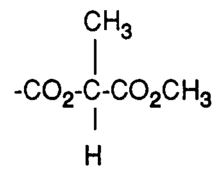 | | $[\alpha]_D$ = -137,78 ($c$=0,8465/$CHCl_3$) — Diasteromere |
| 18 | $-CH_3$ | -CN | $-CO_2-C_2H_5$ | 257 (Zers.) | (-) $[\alpha]_D$ = -220,73 ($c$=0,7113/$CHCl_3$) |
| 19 | $-CH_3$ | -CN | $-CO_2CH_3$ | Schaum | (-) $[\alpha]_D$ = -231,64 ($c$=0,8205/$CHCl_3$) |
| 20 | $-CH_3$ | -CN | $-CO_2-(CH_2)_2-OH$ | Schaum | (-) $[\alpha]_D$ = -208,87 ($c$=0,8455/$CHCl_3$) |
| 21 | $-CH_3$ | -CN | $-CO_2-(CH_2)_2-OCH_3$ | Schaum | (-) $[\alpha]_D$ = -204,27 ($c$=0,716/$CHCl_3$) |
| 22 | 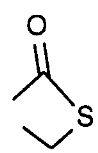 | | $-CO_2-CH(CH_3)_2$ | | |

## Patentansprüche

**1.** 2-Amino-4-heteroaryl-1,4-dihydropyridine der allgemeinen Formel (I)

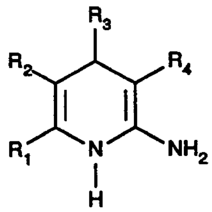

(I)

in welcher

- $R^1$ für geradkettiges oder verzweigtes Alkyl mit bis zu 8 Kohlenstoffatomen steht, das gegebenenfalls durch Hydroxy substituiert ist,
- $R^2$ für geradkettiges oder verzweigtes Alkoxycarbonyl mit bis zu 6 Kohlenstoffatomen steht, das gegebenenfalls durch geradkettiges oder verzweigtes Alkoxy mit bis zu 4 Kohlenstoffatomen substituiert ist, für Nitro, Cyano oder Formyl steht,
  oder
- $R^1$ und $R^2$ gemeinsam einen Ring der Formel

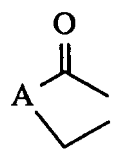

bilden,
worin

- A ein Sauerstoff- oder Schwefelatom, die - $CH_2$- oder -$CH_2CH_2$-Gruppe bedeutet,
- $R^3$ für einen heterocyclischen Arylrest der Formel

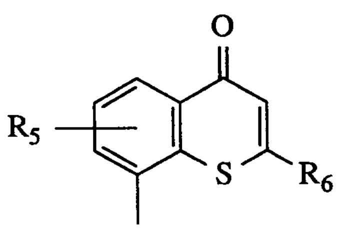 , 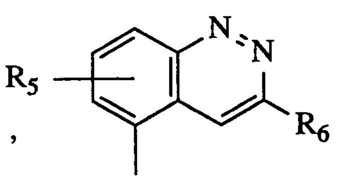 , 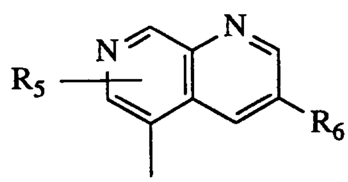

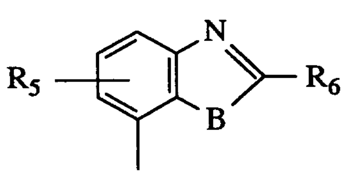 oder 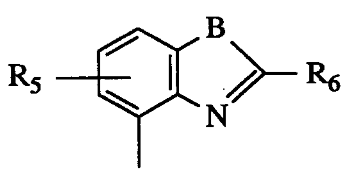

steht,
worin

- B ein Sauerstoff- oder Schwefelatom bedeutet,
- $R^5$ Wasserstoff, Halogen oder geradkettiges oder verzweigtes Alkyl oder Alkoxy mit jeweils bis zu 8 Kohlenstoffatomen bedeutet,
- $R^6$ Aryl mit 6 bis 10 Kohlenstoffatomen bedeutet, das gegebenenfalls bis zu 2-fach gleich oder verschieden durch Halogen, Nitro, Cyano, Trifluormethyl, Trifluormethoxy, Trifluormethylthio, geradkettiges oder verzweigtes Alkyl, Alkoxy oder Alkoxycarbonyl mit jeweils bis zu 8 Kohlenstoffatomen oder Carboxy substituiert ist, oder
  geradkettiges oder verzweigtes Alkyl mit bis zu 12 Kohlenstoffatomen bedeutet, oder
  Cycloalkyl mit 3 bis 8 Kohlenstoffatomen bedeutet, oder
  Pyridyl oder Thienyl bedeutet, das gegebenenfalls durch Halogen substituiert ist,

$R^4$ für eine Gruppe der Formel $-CO-NR^7R^8$, $-CO-D-R^9$ oder $-P(O)(OR^{10})(OR^{11})$ steht, worin

$R^7$ und $R^8$ gleich oder verschieden sind und Wasserstoff oder einen geradkettigen, verzweigten, cyclischen, gesättigten oder ungesättigten Kohlenwasserstoffrest mit bis zu 10 Kohlenstoffatomen bedeuten, der gegebenenfalls durch Cycloalkyl mit 3 bis 8 Kohlenstoffatomen, Halogen, Hydroxy, Cyano oder durch Aryl, Aryloxy, Arylthio mit jeweils 6 bis 10 Kohlenstoffatomen oder durch einen 5- bis 7-gliedrigen, gesättigen oder ungesättigten Heterocyclus mit bis zu 3 Heteroatomen aus der Reihe S, N oder O substituiert ist, wobei die Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O bedeuten, die gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind,
oder

$R^7$ und $R^8$ gemeinsam unter Einbezug des Stickstoffatoms einen 5-bis 8-gliedrigen, gesättigen oder ungesättigten Heterocyclus bilden, der gegebenenfalls durch ein Sauerstoffatom oder durch einen Rest der Formel $S(O)_a$, - CO- oder $-NR^{12}$ unterbrochen ist,
worin

a eine Zahl 0, 1 oder 2 bedeutet,

$R^{12}$ Wasserstoff oder Aryl mit 6 bis 10 Kohlenstoffatomen bedeutet, das gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert ist,
oder
einen cyclischen, geradkettigen oder verzweigten, gesättigten oder ungesättigten Kohlenwasserstoffrest mit bis zu 8 Kohlenstoffatomen bedeutet, der gegebenenfalls durch Hydroxy, Halogen oder durch Aryl mit 6 bis 10 Kohlenstoffatomen oder einen 5- bis 7-gliedrigen, gesättigten oder ungesättigten Heterocyclus mit 3 Heteroatomen aus der Reihe S, N oder O substituiert ist, die ihrerseits bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,

und der Heterocyclus gegebenenfalls durch geradkettiges oder verzweigtes Alkoxy oder Alkylthio mit jeweils bis zu 4 Kohlenstoffatomen, Halogen, Aryl mit 6 bis 10 Kohlenstoffatomen, einen 4- bis 7-gliedrigen, gesättigten oder ungesättigten Heterocyclus mit bis zu 3 Heteroatomen aus der Reihe S, N oder O oder durch geradkettiges oder verzweigtes Alkyl mit bis zu 4 Kohlenstoffatomen substituiert ist, das gegebenenfalls seinerseits durch Aryl mit 6 bis 10 Kohlenstoffatomen substituiert sein kann,

D eine direkte Bindung oder ein Sauerstoffatom bedeutet,

$R^9$ Wasserstoff oder Aryl mit 6 bis 10 Kohlenstoffatomen oder einen 4-bis 7-gliedrigen, gesättigten oder ungesättigten Heterocyclus mit bis zu 3 Heteroatomen aus der Reihe S, N oder O bedeutet, die gegebenenfalls bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind, oder einen cyclischen, geradkettigen oder verzweigten, gesättigten oder ungesättigten Kohlenwasserstoffrest mit bis zu 12 Kohlenstoffatomen bedeutet, der gegebenenfalls bis zu 3-fach gleich oder verschieden durch Sauerstoff oder durch - CO-, -CO-NH-, -O-CO-, -CO-O-, -NH-CO-, $-SO_2-NH-$, $-NH-SO_2-$, - $S(O)_b$- oder $-NR^{13}$ unterbrochen ist,
worin

b die oben angegebene Bedeutung von a hat und mit dieser gleich oder verschieden ist,

$R^{13}$ die oben angegebene Bedeutung von $R^{12}$ hat und mit dieser gleich oder verschieden

ist,

oder der Kohlenwasserstoffrest gegebenenfalls bis zu 3-fach gleich oder verschieden durch Aryliden mit 6 bis 10 Kohlenstoffatomen oder einen cyclischen Rest der Formel

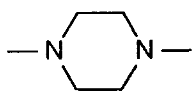 , 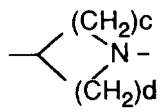 , 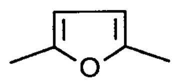 oder 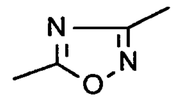

unterbrochen ist,

worin

c und d gleich oder verschieden sind und eine Zahl 1 oder 2 bedeuten,

und worin Aryliden und die Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,

und wobei der Kohlenwasserstoffrest gegebenenfalls bis zu 3-fach gleich oder verschieden durch Cycloalkyl mit 3 bis 8 Kohlenstoffatomen, Halogen, Nitro, Cyano, Hydroxy, - $O-NO_2$, oder durch Aryl, Aryloxy oder Arylthio mit jeweils 6 bis 10 Kohlenstoffatomen oder durch einen 5- bis 7-gliedrigen, gesättigten oder ungesättigen Heterocyclus mit bis zu 3 Heteroatomen aus der Reihe S, N oder O substituiert ist, wobei die Cyclen ihrerseits bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder

der Kohlenwasserstoffrest durch eine Gruppe der Formel - $CO_2$-$R^{14}$, -CONR$^{15}$R$^{16}$ oder -NR$^{17}$R$^{18}$ substituiert ist,

worin

$R^{14}$ die oben angegebene Bedeutung von $R^{12}$ hat und mit dieser gleich oder verschieden ist

und

$R^{15}$, $R^{16}$, $R^{17}$ und $R^{18}$ die oben angegebene Bedeutung von $R^7$ und $R^8$ haben und mit diesen gleich oder verschieden sind,

$R^{10}$ und $R^{11}$ gleich oder verschieden sind und geradkettiges oder verzweigtes Alkyl mit bis zu 8 Kohlenstoffatomen bedeuten,

oder gemeinsam unter Einbezug der Sauerstoffatome einen 5-bis 7-gliedrigen, gesättigen Carbocyclus bilden,

und deren Salze,

**2.** Verbindungen der allgemeinen Formel (I) gemäß Anspruch 1

in welcher

$R^1$ für geradkettiges oder verzweigtes Alkyl mit bis zu 6 Kohlenstoffatomen steht, das gegebenenfalls durch Hydroxy substituiert ist,

$R^2$ für geradkettiges oder verzweigtes Alkoxycarbonyl mit bis zu 4 Kohlenstoffatomen steht, das gegebenenfalls durch geradkettiges oder verzweigtes Alkoxy mit bis zu 3 Kohlenstoffatomen substituiert ist, für Nitro, Cyano oder Formyl steht,

oder

$R^1$ und $R^2$ gemeinsam einen Ring der Formel

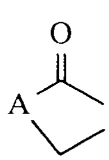

bilden,

worin

A ein Sauerstoff- oder Schwefelatom, die - $CH_2$- oder -$CH_2CH_2$-Gruppe bedeutet,

$R^3$ für einen heterocyclischen Rest der Formel

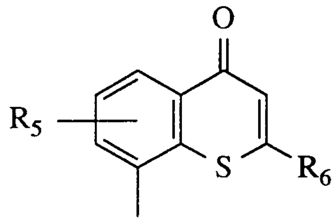
,
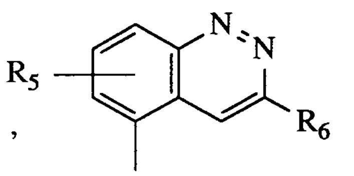
,
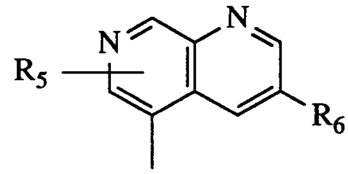

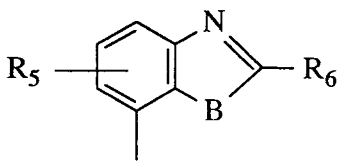
oder
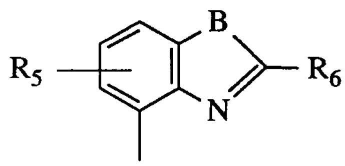

steht,

worin

B ein Sauerstoff- oder Schwefelatom bedeutet,

$R^5$ Wasserstoff, Fluor, Chlor oder geradkettiges oder verzweigtes Alkyl oder Alkoxy mit jeweils bis zu 2 Kohlenstoffatomen bedeutet,

$R^6$ Phenyl bedeutet, das gegebenenfalls durch Fluor, Chlor, Nitro, Cyano, Trifluormethyl oder durch geradkettiges oder verzweigtes Alkyl oder Alkoxy mit jeweils bis zu 6 Kohlenstoffatomen substituiert ist, oder
geradkettiges oder verzweigtes Alkyl mit bis zu 10 Kohlenstoffatomen bedeutet, oder Cyclopropyl, Cyclohexyl oder Cyclopentyl bedeutet, oder Pyridyl oder Thienyl bedeutet, das gegebenenfalls durch Fluor, Chlor oder Brom substituiert ist,

$R^4$ für eine Gruppe der Formel -CO-$NR^7R^8$, -CO-D-$R^9$ oder -P(O)($OR^{10}$)($OR^{11}$) steht,
worin

$R^7$ und $R^8$ gleich oder verschieden sind und Wasserstoff oder einen geradkettigen, verzweigten, cyclischen, gesättigten oder ungesättigten Kohlenwasserstoffrest mit bis zu 6 Kohlenstoffatomen bedeuten, der gegebenenfalls durch Cyclopropyl, Cyclopentyl, Cyclohexyl, Fluor, Chlor, Brom, Hydroxy oder durch Phenyl oder Pyridyl substituiert ist, wobei die Cyclen ihrerseits durch Fluor, Chlor, Cyano oder durch geradkettiges oder verzweigtes Alkyl, Alkoxy, Alkylthio, Alkoxycarbonyl mit jeweils bis zu 3 Kohlenstoffatomen, Trifluormethyl oder Trifluormethoxy substituiert sein können, oder
Phenyl oder Pyridyl bedeuten, die gegebenenfalls durch Fluor, Chlor oder durch geradkettiges oder verzweigtes Alkyl, Alkoxy, Alkylthio, Alkoxycarbonyl mit jeweils bis zu 4 Kohlenstoffatomen, Trifluormethyl oder Trifluormethoxy substituiert sind,
oder

$R^7$ und $R^8$ gemeinsam unter Einbezug des Stickstoffatoms einen 5-bis 7-gliedrigen, gesättigen oder ungesättigten Heterocyclus bilden, oder gegebenenfalls durch ein Sauerstoffatom oder durch einen Rest der Formel $S(O)_a$, - CO- oder -$NR^{12}$ unterbrochen sein kann,
worin

a eine Zahl 0, 1 oder 2 bedeutet,

$R^{12}$ Wasserstoff oder Phenyl bedeutet, oder
einen cyclischen, geradkettigen oder verzweigten, gesättigten oder ungesättigten Kohlenwasserstoffrest mit bis zu 6 Kohlenstoffatomen bedeutet, der gegebenenfalls durch Fluor, Chlor, oder durch Phenyl oder Pyridyl substituiert ist, die ihrerseits durch Fluor, Chlor, Methyl, Ethyl, Methoxy, Ethoxy, Trifluormethyl oder Trifluormethoxy substituiert sein können,

und der Heterocyclus gegebenenfalls durch geradkettiges oder verzweigtes Alkoxy oder Alkylthio mit jeweils bis zu 3 Kohlenstoffatomen, Fluor, Chlor, Phenyl oder Pyridyl oder durch verzweigtes Alkyl mit bis zu 4 Kohlenstoffatomen oder Benzyl

substituiert ist,

D eine direkte Bindung oder ein Sauerstoffatom bedeutet,

$R^9$ Wasserstoff, Phenyl oder Pyridyl bedeutet, die gegebenenfalls durch Fluor, Chlor oder durch geradkettiges oder verzweigtes Alkyl, Alkoxy oder Alkylthio mit jeweils bis zu 3 Kohlenstoffatomen, Trifluormethyl oder Trifluormethoxy substituiert sind, oder einen cyclischen, geradkettigen oder verzweigten, gesättigten oder ungesättigten Kohlenwasserstoffrest mit bis zu 10 Kohlenstoffatomen bedeutet, der gegebenenfalls bis zu 2-fach gleich oder verschieden durch Sauerstoff oder durch - CO-, -O-CO-, -NH-CO-, $-SO_2$-NH-, -NH-$SO_2$-, -S(O)$_b$- oder -$NR^{13}$ unterbrochen ist,
worin

b die oben angegebene Bedeutung von a hat und mit dieser gleich oder verschieden ist,

$R^{13}$ die oben angegebene Bedeutung von $R^{12}$ hat und mit dieser gleich oder verschieden ist,

oder der Kohlenwasserstoffrest gegebenenfalls bis zu 2-fach gleich oder verschieden durch Phenyliden oder einen cyclischen Rest der Formel

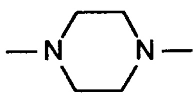 , 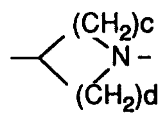 , 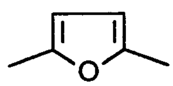 oder 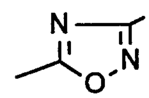

unterbrochen ist,
worin

c und d gleich oder verschieden sind und eine Zahl 1 oder 2 bedeuten,
und wobei der Kohlenwasserstoffrest gegebenenfalls bis zu 2-fach gleich oder verschieden durch Cyclopropyl, Cyclopentyl, Cyclohexyl, Fluor, Chlor, Nitro, Cyano, Hydroxy, - O-$NO_2$, geradkettiges oder verzweigtes Alkylthio, Alkoxy oder Acyloxy mit jeweils bis zu 6 Kohlenstoffatomen oder durch Phenyl, Phenoxy, Phenylthio oder Pyridyl substituiert ist, wobei die Cyclen ihrerseits bis zu 2-fach gleich oder verschieden durch Fluor, Chlor, Cyano oder durch geradkettiges oder verzweigtes Alkyl, Alkoxy, Alkylthio, Alkoxycarbonyl mit jeweils mit bis zu 4 Kohlenstoffatomen, Trifluormethyl oder Trifluormethoxy substituiert sind, oder
der Kohlenwasserstoffrest durch eine Gruppe der Formel - $CO_2$-$R^{14}$, -CON$R^{15}R^{16}$ oder -N$R^{17}R^{18}$ substituiert ist,
worin

$R^{14}$ die oben angegebene Bedeutung von $R^{12}$ hat und mit dieser gleich oder verschieden ist
und

$R^{15}$, $R^{16}$, $R^{17}$ und $R^{18}$ die oben angegebene Bedeutung von $R^7$ und $R^8$ haben und mit diesen gleich oder verschieden sind,

$R^{10}$ und $R^{11}$ gleich oder verschieden sind und geradkettiges oder verzweigtes Alkyl mit bis zu 6 Kohlenstoffatomen bedeuten
oder

$R^{10}$ und $R^{11}$ gemeinsam unter Einbezug des Sauerstoffatoms einen 6-gliedrigen gesättigten Carbocyclus bilden,

und deren Salze,

**3.** Verbindungen der allgemeinen Formel (I) gemäß Anspruch 1
in welcher

$R^1$ für geradkettiges oder verzweigtes Alkyl mit bis zu 4 Kohlenstoffatomen steht, das gegebenenfalls durch Hydroxy substituiert ist,

$R^2$ für geradkettiges oder verzweigtes Alkoxycarbonyl mit bis zu 3 Kohlenstoffatomen oder Methoxyethoxycarbonyl steht, oder
für Nitro, Cyano oder Formyl steht,
oder

$R^1$ und $R^2$ gemeinsam einen Ring der Formel

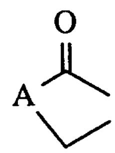

bilden,
worin

A ein Sauerstoff- oder Schwefelatom, die - $CH_2$- oder -$CH_2CH_2$-Gruppe bedeutet,

$R^3$ für einen heterocyclischen Rest der Formel

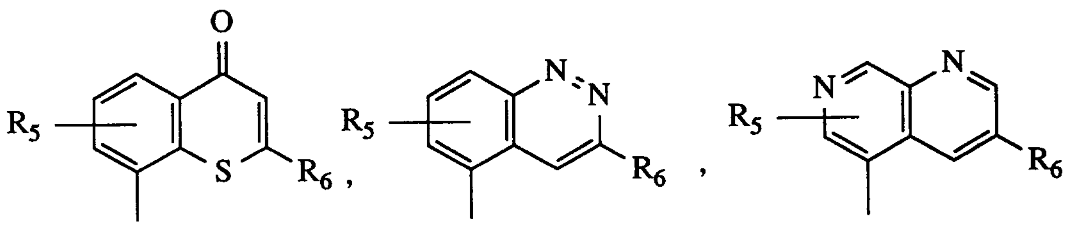

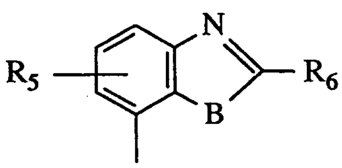 oder 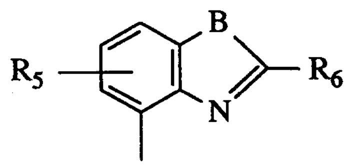

steht,
worin

| | |
|---|---|
| B | ein Sauerstoff- oder Schwefelatom bedeutet, |
| $R^5$ | Wasserstoff, Chlor oder Methyl bedeutet, |
| $R^6$ | Phenyl bedeutet, das gegebenenfalls durch Fluor, Chlor, Nitro, Trifluormethyl oder durch geradkettiges oder verzweigtes Alkyl oder Alkoxy mit jeweils bis zu 4 Kohlenstoffatomen substituiert ist, oder<br>geradkettiges oder verzweigtes Alkyl mit bis zu 8 Kohlenstoffatomen bedeutet, oder<br>Cyclopropyl, Cyclohexyl oder Cyclopentyl bedeutet, oder Pyridyl oder Thienyl bedeutet, das gegebenenfalls durch Fluor, Chlor oder Brom substituiert ist, |
| $R^4$ | für eine Gruppe der Formel -CO-$NR^7R^8$ oder -CO-D-$R^9$ steht,<br>worin |
| $R^7$ und $R^8$ | gleich oder verschieden sind und Wasserstoff oder Phenyl oder einen geradkettigen, verzweigten, cyclischen, gesättigten oder ungesättigten Kohlenwasserstoffrest mit bis zu 6 Kohlenstoffatomen bedeuten, der gegebenenfalls durch Phenyl substituiert ist, das seinerseits durch Fluor, Chlor, Methyl, Ethyl, Methoxy oder Ethoxy substituiert sein kann, |
| D | eine direkte Bindung oder ein Sauerstoffatom bedeutet, |
| $R^9$ | Wasserstoff oder Phenyl bedeutet, oder<br>einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel oder durch einen Rest der Formel - O-CO-, -NH-CO- oder -$NR^{13}$ unterbrochen ist,<br>worin |
| $R^{13}$ | Wasserstoff oder geradkettiges oder verzweigtes Alkyl mit bis zu 4 Kohlenstoffatomen bedeutet, das gegebenenfalls durch Phenyl substituiert |

ist, oder Phenyl bedeutet,

und wobei der Kohlenwasserstoffrest gegebenenfalls durch Cyclopropyl, Cyclopentyl, Cyclohexyl, Fluor, Chlor, Cyano, Hydroxy, Phenyl, Phenoxy, Phenylthio oder Pyridyl substituiert ist, oder durch eine Gruppe der Formel - $CO_2$-$R^{14}$, -CONR$^{15}$R$^{16}$ oder -NR$^{17}$R$^{18}$ substituiert ist,

worin

$R^{14}$ die oben angegebene Bedeutung von $R^{13}$ hat und mit dieser gleich oder verschieden ist

und

$R^{15}$, $R^{16}$, $R^{17}$ und $R^{18}$ die oben angegebene Bedeutung von $R^7$ und $R^8$ haben und mit diesen gleich oder verschieden sind,

und deren Salze.

**4.** Verfahren zur Herstellung von Verbindungen der allgemeinen Formel (I)

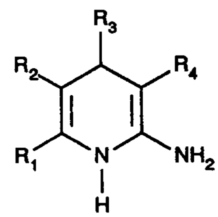

(I)

in welcher

$R^1$ für geradkettiges oder verzweigtes Alkyl mit bis zu 8 Kohlenstoffatomen steht, das gegebenenfalls durch Hydroxy substituiert ist,

$R^2$ für geradkettiges oder verzweigtes Alkoxycarbonyl mit bis zu 6 Kohlenstoffatomen steht, das gegebenenfalls durch geradkettiges oder verzweigtes Alkoxy mit bis zu 4 Kohlenstoffatomen substituiert ist, für Nitro, Cyano oder Formyl steht,

oder

$R^1$ und $R^2$ gemeinsam einen Ring der Formel

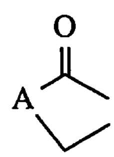

bilden,

worin

A ein Sauerstoff- oder Schwefelatom, die - $CH_2$- oder -$CH_2CH_2$-Gruppe bedeutet,

$R^3$ für einen heterocyclischen Arylrest der Formel

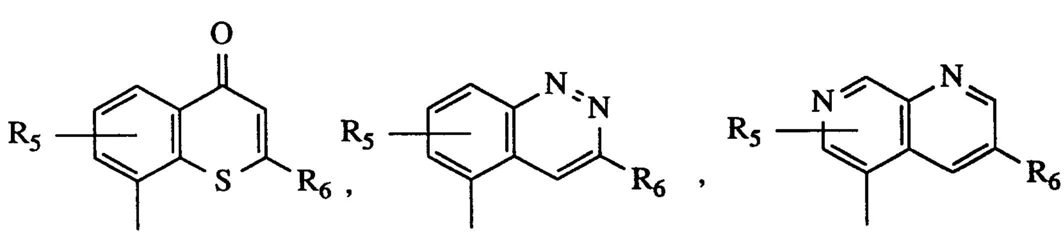

, ,

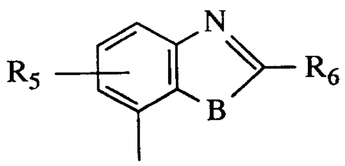

oder

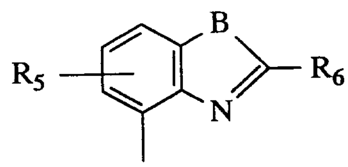

steht,

worin

B ein Sauerstoff- oder Schwefelatom bedeutet,

$R^5$ Wasserstoff, Halogen oder geradkettiges oder verzweigtes Alkyl oder Alkoxy mit jeweils bis zu 8 Kohlenstoffatomen bedeutet,

$R^6$ Aryl mit 6 bis 10 Kohlenstoffatomen bedeutet, das gegebenenfalls bis zu 2-fach gleich oder verschieden durch Halogen, Nitro, Cyano, Trifluormethyl, Trifluormethoxy, Trifluormethylthio, geradkettiges oder verzweigtes Alkyl, Alkoxy oder Alkoxycarbonyl mit jeweils bis zu 8 Kohlenstoffatomen oder Carboxy substituiert ist, oder
geradkettiges oder verzweigtes Alkyl mit bis zu 12 Kohlenstoffatomen bedeutet, oder Cycloalkyl mit 3 bis 8 Kohlenstoffatomen bedeutet, oder Pyridyl oder Thienyl bedeutet, das gegebenenfalls durch Halogen substituiert ist,

$R^4$ für eine Gruppe der Formel $-CO-NR^7R^8$, $-CO-D-R^9$ oder $-P(O)(OR^{10})(OR^{11})$ steht,
worin

$R^7$ und $R^8$ gleich oder verschieden sind und Wasserstoff oder einen geradkettigen, verzweigten, cyclischen, gesättigten oder ungesättigten Kohlenwasserstoffrest mit bis zu 10 Kohlenstoffatomen bedeuten, der gegebenenfalls durch Cycloalkyl mit 3 bis 8 Kohlenstoffatomen, Halogen, Hydroxy, Cyano oder durch Aryl, Aryloxy, Arylthio mit jeweils 6 bis 10 Kohlenstoffatomen oder durch einen 5- bis 7-gliedrigen, gesättigen oder ungesättigten Heterocyclus mit bis zu 3 Heteroatomen aus der Reihe S, N oder O substituiert ist, wobei die Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder
Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O bedeuten, die gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind,
oder

$R^7$ und $R^8$ gemeinsam unter Einbezug des Stickstoffatoms einen 5-bis 8-gliedrigen, gesättigen oder ungesättigten Heterocyclus bilden, der gegebenenfalls durch ein Sauerstoffatom oder durch einen Rest der Formel $S(O)_a$, - CO- oder $-NR^{12}$ unterbrochen ist,
worin

a eine Zahl 0, 1 oder 2 bedeutet,

$R^{12}$ Wasserstoff oder Aryl mit 6 bis 10 Kohlenstoffatomen bedeutet, das gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert ist, oder
einen cyclischen, geradkettigen oder verzweigten, gesättigten oder ungesättigten Kohlenwasserstoffrest mit bis zu 8 Kohlenstoffatomen bedeutet, der gegebenenfalls durch Hydroxy, Halogen oder durch Aryl mit 6 bis 10 Kohlenstoffatomen oder einen 5- bis 7-gliedrigen, gesättigten oder ungesättigten Heterocyclus mit 3 Heteroatomen aus der Reihe S, N oder O substituiert ist, die ihrerseits bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,

und der Heterocyclus gegebenenfalls durch geradkettiges oder verzweigtes Alkoxy oder Alkylthio mit jeweils bis zu 4 Kohlenstoffatomen, Halogen, Ary lmit 6 bis 10

Kohlenstoffatomen, einen 4- bis 7-gliedrigen, gesättigten oder ungesättigten Heterocyclus mit bis zu 3 Heteroatomen aus der Reihe S, N oder O oder durch geradkettiges oder verzweigtes Alkyl mit bis zu 4 Kohlenstoffatomen substituiert ist, das gegebenenfalls seinerseits durch Aryl mit 6 bis 10 Kohlenstoffatomen substituiert sein kann,

D eine direkte Bindung oder ein Sauerstoffatom bedeutet,

$R^9$ Wasserstoff oder Aryl mit 6 bis 10 Kohlenstoffatomen oder einen 4-bis 7-gliedrigen, gesättigten oder ungesättigten Heterocyclus mit bis zu 3 Heteroatomen aus der Reihe S, N oder O bedeutet, die gegebenenfalls bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind, oder
einen cyclischen, geradkettigen oder verzweigten, gesättigten oder ungesättigten Kohlenwasserstoffrest mit bis zu 12 Kohlenstoffatomen bedeutet, der gegebenenfalls bis zu 3-fach gleich oder verschieden durch Sauerstoff oder durch - CO-, -CO-NH-, -O-CO-, -CO-O-, -NH-CO-, $-SO_2-NH-$, $-NH-SO_2-$, - $S(O)_b$-oder $-NR^{13}$ unterbrochen ist,
worin

b die oben angegebene Bedeutung von a hat und mit dieser gleich oder verschieden ist,

$R^{13}$ die oben angegebene Bedeutung von $R^{12}$ hat und mit dieser gleich oder verschieden ist,

oder der Kohlenwasserstoffrest gegebenenfalls bis zu 3-fach gleich oder verschieden durch Aryliden mit 6 bis 10 Kohlenstoffatomen oder einen cyclischen Rest der Formel

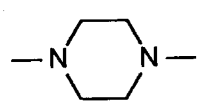 , 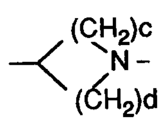 , 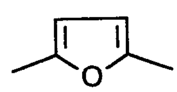 oder 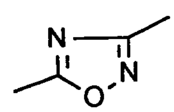

unterbrochen ist,
worin

c und d gleich oder verschieden sind und eine Zahl 1 oder 2 bedeuten,

und worin Aryliden und die Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,

und wobei der Kohlenwasserstoffrest gegebenenfalls bis zu 3-fach gleich oder verschieden durch Cycloalkyl mit 3 bis 8 Kohlenstoffatomen, Halogen, Nitro, Cyano, Hydroxy, - $O-NO_2$, oder durch Aryl, Aryloxy oder Arylthio mit jeweils 6 bis 10 Kohlenstoffatomen oder durch einen 5- bis 7-gliedrigen, gesättigten oder ungesättigen Heterocyclus mit bis zu 3 Heteroatomen aus der Reihe S, N oder O substituiert ist, wobei die Cyclen ihrerseits bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder
der Kohlenwasserstoffrest durch eine Gruppe der Formel - $CO_2-R^{14}$, $-CONR^{15}R^{16}$ oder $-NR^{17}R^{18}$ substituiert ist,
worin

$R^{14}$ die oben angegebene Bedeutung von $R^{12}$ hat und mit dieser gleich oder verschieden ist
und

$R^{15}$, $R^{16}$, $R^{17}$ und $R^{18}$ die oben angegebene Bedeutung von $R^7$ und $R^8$ haben und mit diesen gleich oder verschieden sind,

$R^{10}$ und $R^{11}$ gleich oder verschieden sind und geradkettiges oder verzweigtes Alkyl mit bis zu 8 Kohlenstoffatomen bedeuten,

oder gemeinsam unter Einbezug der Sauerstoffatome einen 5-bis 7-gliedrigen, gesättigen Carbocyclus bilden,

und deren Salze, dadurch gekennzeichnet, daß man

[A] entweder Aldehyde der allgemeinen Formel (II)

$R^3$-CHO (II)

in welcher

$R^3$ die oben angegebene Bedeutung hat,

direkt mit Verbindungen der allgemeinen Formel (III)

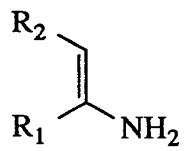

(III)

in welcher

$R^1$ und $R^2$ die oben angegebene Bedeutung haben,

und Verbindungen der tautomeren Formeln (IV) und (IVa)

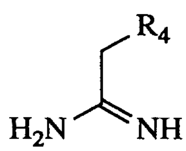

(IV)

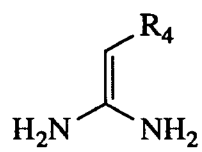

(IVa)

in welcher

$R^4$ die oben angegebene Bedeutung hat,

in inerten Lösemittteln bei Temperaturen zwischen 10 °C und 150 °C umsetzt,

oder

[B] Ylidenverbindungen der allgemeinen Formel (V)

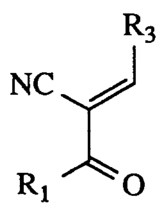

(V)

in welcher

$R^1$ und $R^3$ die oben angegebene Bedeutung haben,

mit Verbindungen der allgemeinen Formel (VI) bzw. (VIa)

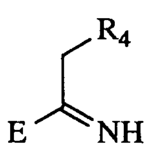

(VI)

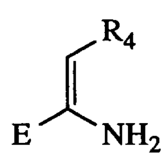

(VIa)

in welcher

$R^4$ die oben angegebene Bedeutung hat, und

E für die Aminogruppe oder für $C_1$-$C_4$-Alkoxy steht,

gegebenenfalls in Anwesenheit von inerten organischen Lösemitteln bei Temperaturen von 10 °C bis 150 °C umsetzt, wobei für den Fall, daß E für $C_1$-$C_4$-Alkoxy steht, Ammoniumsalze zugegeben zugegeben werden.

**5.** Aldehyde der allgemeinen Formel (II)

$R^3$-CHO (II)

in welcher
$R^3$ für einen Rest der Formel

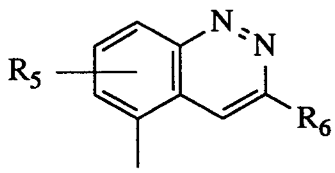

oder

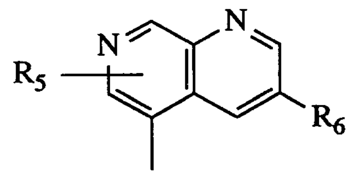

steht,
worin
$R^5$ Wasserstoff, Halogen oder geradkettiges oder verzweigtes Alkyl oder Alkoxy mit jeweils bis zu 8 Kohlenstoffatomen bedeutet, und
$R^6$ Aryl mit 6 bis 10 Kohlenstoffatomen bedeutet, das gegebenenfalls bis zu 2-fach gleich oder verschieden durch Halogen, Nitro, Cyano, Trifluormethyl, Trifluormethoxy, Trifluormethylthio, geradkettiges oder verzweigtes Alkyl, Alkoxy oder Alkoxycarbonyl mit jeweils bis zu 8 Kohlenstoffatomen oder Carboxy substituiert ist, oder
geradkettiges oder verzweigtes Alkyl mit bis zu 12 Kohlenstoffatomen bedeutet, oder
Cycloalkyl mit 3 bis 8 Kohlenstoffatomen bedeutet, oder Pyridyl oder Thienyl bedeutet, das gegebenenfalls durch Halogen substituiert ist.

**6.** Verfahren zur Herstellung von Aldehyden der allgemeinen Formel (II) gemäß Anspruch 5, dadurch gekennzeichnet, daß man
a) substituierte Pyridine der allgemeinen Formel (VII)

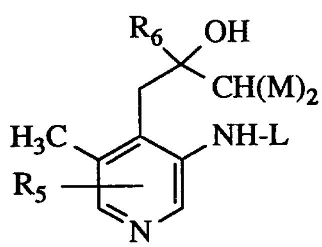

(VII),

in welcher
$R^5$, $R^6$ die oben angegebene Bedeutung haben,
L für eine Amino-Schutzgruppe steht
und
M für geradkettiges oder verzweigtes Alkoxy mit bis zu 4 Kohlenstoffatomen steht,
zunächst mit Protonensäuren umsetzt und mit anschließender Hydrierung zu Verbindungen der allgemeinen Formel (VIII)

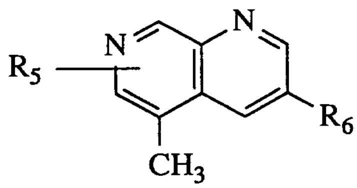

(VIII),

in welcher

$R^5$ und $R^6$ die oben angegebene Bedeutung haben,

in inerten Lösemitteln cyclisiert und in einem letzten Schritt in einem organischen Lösemittel die Methylgruppe oxidiert,

und im Fall, daß $R^3$ für den Rest der Formel

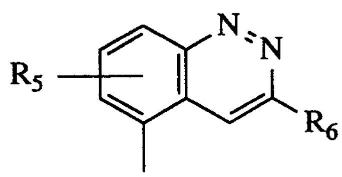

steht,

b) Verbindungen der allgemeinen Formel (IX)

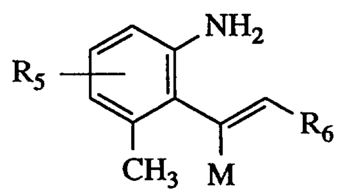

(IX),

in welcher

$R^5$, $R^6$ und M die oben angegebene Bedeutung haben,

über die diazotierte Stufe ($NH_2$ -> $N_2^+$) zu Verbindungen der allgemeinen Formel (X)

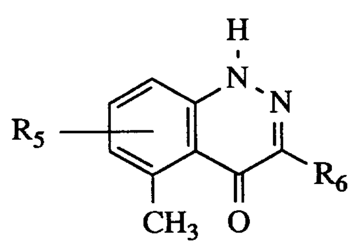

(X),

in welcher

$R^5$ und $R^6$ die oben angegebene Bedeutung haben,

cyclisiert, in einem zweiten Schritt mit $PCl_5/POCl_3$ in Verbindungen der allgemeinen Formel (XI)

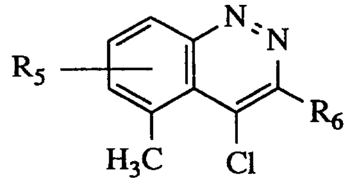

(XI),

in welcher

$R^5$ und $R^6$ die oben angegebene Bedeutung haben

überführt, hydriert und abschließend die Methyl-Gruppe in inerten Lösemitteln oxidiert.

**7.** Verbindungen der allgmeinen Formel (I) gemäß Anspruch 1 zur Verwendung bei der Behandlung von Herz-Kreislauferkrankungen.

**8.** Arzneimittel enthaltend mindestens eine Verbindung der allgemeinen Formel (I) gemäß Anspruch 1.

**9.** Verfahren zur Herstellung von Arzneimitteln, dadurch gekennzeichnet, daß man mindestens eine Verbindung der allgemeinen Formel (I) gemäß Anspruch 1 gegebenenfalls mit üblichen Hilfs- und Trägerstoffen in eine geeignete Applikationsform überführt.

**10.** Verwendung von Verbindungen der allgemeinen Formel (I) gemäß Anspruch 1 in Arzneimitteln mit positiv inotroper Wirkung.

Europäisches Patentamt

# EUROPÄISCHER RECHERCHENBERICHT

Nummer der Anmeldung

EP 94105777.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
|---|---|---|---|
| D,A | EP - A - 0 515 940 (BAYER AG) * Ansprüche 1,5,8-10 * -- | 1,4, 7-10 | C 07 D 401/04<br>C 07 D 409/04<br>C 07 D 413/04<br>C 07 D 417/04<br>C 07 D 471/04<br>A 61 K 31/44<br>A 61 K 31/50 |
| A | DE - A - 2 639 257 (BAYER AG) * Ansprüche 1,4; Beispiel 2 * -- | 1,8,9 | |
| A | US - A - 3 946 026 (MEYER) * Anspruch 1 * -- | 1 | |
| A,P | EP - A - 0 538 690 (BAYER AG) * Ansprüche 1,5-8 * -- | 1,4, 7-10 | |
| A | CHEMICAL ABSTRACTS, Band 88, Nr. 17, 24. April 1978, Columbus, Ohio, USA H. MEYER et al. "Dihydropyridines, III. Synthesis of 2-amino-dihydropyridines via Michael addition." Seite 525, Spalte 1, Nr. 120 947c; & Justus Liebigs Ann. Chem. 1977, (11-12), 1895-908 (Ger). -- | 1 | RECHERCHIERTE SACHGEBIETE (Int. Cl.⁵)<br>C 07 D 401/00<br>C 07 D 409/00<br>C 07 D 413/00<br>C 07 D 417/00<br>C 07 D 471/00<br>A 61 K 31/00 |
| P,X | CHEMICAL ABSTRACTS, Band 118, Nr. 23, 7. Juni 1993, Columbus, Ohio, USA A. Straub "Regioselective metalation of ortho--aminopicolines using the pivaloyl group as a directing group: synthesis of naphthyridines." Seite 987, Spalte 1, Nr. 233 917z; | 5 | |

Der vorliegende Recherchenbericht wurde für alle Patentansprüche erstellt.

| Recherchenort | Abschlußdatum der Recherche | Prüfer |
|---|---|---|
| WIEN | 21-06-1994 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82

Europäisches Patentamt

**EUROPÄISCHER RECHERCHENBERICHT**

Nummer der Anmeldung



EP 94105777.0

**EINSCHLÄGIGE DOKUMENTE**

| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
|---|---|---|---|
| | & Synth Commun. 1993, 23(3), 365-72 (Eng). -- | | |
| A | CHEMICAL ABSTRACTS, Band 108, Nr. 13, 28. März 1988, Columbus, Ohio, USA BARTON J.W. et al. "Cinnolines. Part 1. Widman--Stoermer reactions of functionalized 2-phenylbut-2--enes to give cinnolinecarb--aldehydes." Seite 622, Spalte 1, Nr. 112 361y; & J. Chem. Soc., Perkin Trans.1 1987, (7), 1541-5 (Eng). -- | 5 | |
| A | CHEMICAL ABSTRACTS, Band 100, Nr. 11, 12. März 1984, Columbus, Ohio, USA METH-COHN O. et al. "Pyridine annelation of o-methylarylcarboxylic acids with Vilsmeier reagents." Seite 532, Spalte 2, Nr. 85 564t; & Tetrahedron Lett. 1983, 24(42), 4607-10 (Eng). ---- | 5 | RECHERCHIERTE SACHGEBIETE (Int. Cl.⁵) |

Der vorliegende Recherchenbericht wurde für alle Patentansprüche erstellt.

| Recherchenort | Abschlußdatum der Recherche | Prüfer |
|---|---|---|
| WIEN | 21-06-1994 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 62